# EUROPEAN PATENT APPLICATION

(11) **EP 3 238 660 A1**
(43) Date of publication of application: **01.11.2017**
(21) Application number: 17168705.6
(22) Date of filing: 28.04.2017
(51) Int. Cl.: A61F 2/16, A61F 9/008

(54) **APPARATUS TO MODIFY ACCOMMODATING INTRAOCULAR LENS**

(30) Priority: 29.04.2016 NL 2016704
(71) Applicant: Akkolens International B.V., 4836 BA Breda (NL)
(72) Inventor: SIMONOV, Aleksey Nikolaevich, 2497 DV Den Haag (NL); ROMBACH, Michiel Christiaan, 4836 BA Breda (NL)
(74) Representative: Patentwerk B.V.

(57) **Abstract**

This document describes an apparatus and a method to adjust mechanical properties of an intraocular lens comprising at least two haptics and at least one optical element, with the apparatus comprising at least one laser light source adapted to provide inscription of a pattern in the lens and a digital control unit adapted to control the laser light.

## Description

This document discloses an apparatus for adjustment of mechanical properties of any accommodating lens, for example, but not restricted hereto, hydrophilic acrylic lenses, for example, as disclosed in WO2005084587 and US2015342728 and related documents, which will be put forward to illustrate the apparatus and methods disclosed in this document. These lenses comprise at least two optical elements, each element with at least one free-form optical surface, which surfaces, in combination, form a lens of variable power which power depends on the relative position of the optical elements in a direction perpendicular to the optical axis.

An apparatus and methods adapted to provide adjustment of optical properties at least one optical component of hydrophilic acrylic intraocular lenses, by laser light, is known from prior-art documents, for example, US2014/0135920A1 and related documents.

The present invention discloses an apparatus comprising at least one laser light source adapted to provide inscription of a pattern in at least one mechanical component of the lens and a digital control unit connected to the laser light source and adapted to control the laser light source such that the inscription pattern is inscribed in the lens. Said inscription pattern is designed to provide at least one adjustment of at least one mechanical property of at least one said mechanical component of the lens. A mechanical component of an intraocular lens is generally referred to as a haptic, generally a plastic side strut, designed to hold the lens, the optics of the lens, in the proper place in the eye.
So, the invention provides a method to adjust mechanical properties of a haptic of an accommodating hydrophilic acrylic intraocular lens by irradiating an inscription pattern in the lens by a laser beam under control of a digital control unit.
Also, the optical properties of the lens in the eye can be adjusted by adjustment of the position in the eye by adjustment of at least one haptic. For example, focusing of the lens can be changed by movement of the lens along the optical axis, or, tilting of the lens can be adjusted by asymmetrical adjustment of for example, only one of a set of two haptics, or, alternatively, the mechanical properties of the lens can be adjusted by adjustment of the optical component in case the optical component is a shape changing optical component as in, for example,

The first preferred embodiment provides an apparatus of the kind referred to above, wherein the laser light source is adapted to inscribe the pattern in at least one of the haptics of the lens and that the digital control unit is adapted to control the laser light source such that the inscription pattern changes the mechanical properties of the lens. Such change can be achieved by, for example, change the size or shape of the haptic or, alternatively, change the mechanical property of the materials.

The apparatus can be adapted to adjust the properties of an intra ocular lens during manufacturing, prior or during implantation surgery, but most importantly, adjust the properties of an intraocular lens inside the eye in postoperative adjustment procedures.

Preferably the laser light source is a femto-second laser light source, which light source can be adapted to cause effects as local ablation, local swelling, local inscription, or local cutting, or a combination of such effects. The embodiment can provide the feature that the apparatus comprises a diagnostic unit is adapted to provide real-time input for the digital control unit.
The diagnostic unit can comprise a light source and a detector to measure the optical properties of the lens in the eye so that the effects of adjustments can be measured during the adjustment procedures.

The diagnostic unit can comprise a mirror, or, alternatively, beam splitter, located in the optical path between the light source, the detector respectively and the lens, and that the mirror is located in the optical path between the laser light source and the lens. This embodiment allows inscribing the pattern simultaneously with the measurement of the optical properties of the lens.

The apparatus provides a change in mechanical properties at the section which bends and subsequently relaxes during the accommodative process in the eye. The apparatus comprises a diagnostic unit for diagnosis to provide input for a digital
control unit, for calculating the inscription pattern and driving and targeting the laser,
and a laser unit for delivering the inscription pattern onto or into to the target.

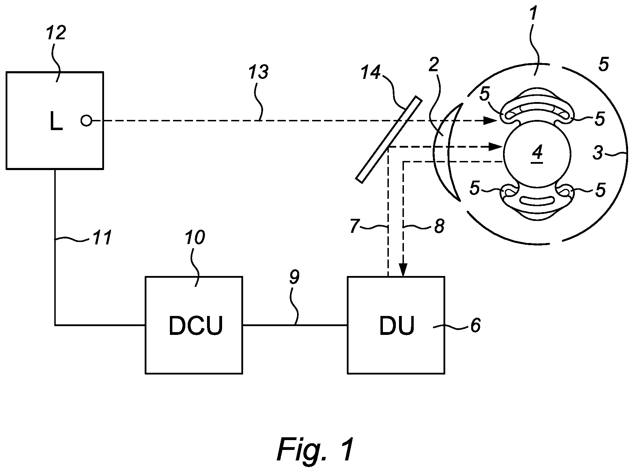

Figure 1 depicts a schematic view of the apparatus;
Figure 2 shows a diagram of an intraocular lens, both in a plan view and in a side view; and
Figure 3, a perspective view of the intraocular lens depicted in figure 2.

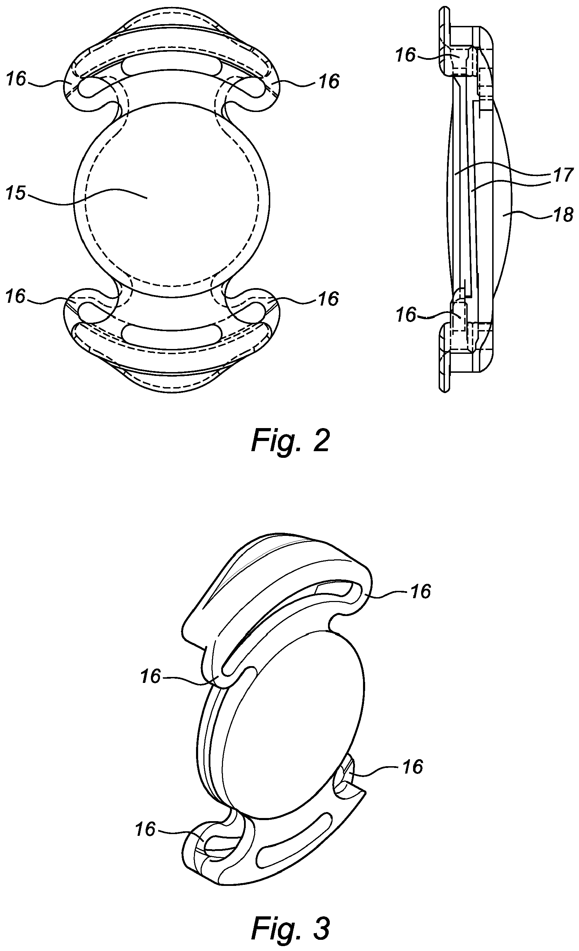

Figure 2 shows an apparatus according to the invention with a human eye 1, the cornea 2, and retina 3, and the intraocular lens 4, with the elastic wings of the haptics 5, and a diagnostic
unit, for example, any OCT apparatus 6, with an outgoing illumination light ray 7, generated by a light source and an incoming reflected light ray 8 for diagnosis 8, with the signal from the diagnostic unit 6 transferred to a digital control unit 10 via a connection 9.
The control unit 10 converts the incoming signal via connection 9 into a driving signal for the femto-second laser unit 12, via connection 11, which laser unit 12 generates laser pulses 13, onto or into, the haptic wing to adjust the shape or the elastic properties or a combination
thereof. It is also possible that the optical elements themselves are treated by the laser. A beam splitter or semi transparent mirror 14, is added to allow the laser unit to operate simultaneously with the diagnostic unit 10.

Figure 2 depicts an intraocular lens 15, as disclosed in WO2005084587 and in US2015342728, with four haptic wings 16, which wings can be treated by laser to provide
desired adjustments.

Figure 3 shows a perspective view of the lens depicted in Fig.2. The laser can provide local ablation, local cutting or a combination of ablation, swelling and/or cutting of the hydrophilic material. The first embodiment of the apparatus comprises at least one laser which is driven by signals from at least one a digital control unit which, based on imaging input from a diagnostic unit, for example input from an Optical Coherence Tomograph (OCT) which can be 3D-spectral domain OCT, or, time domain OCT, or, a combination of 3D-spectral domain OCT and time domain OCT.

The digital control unit provides the laser with a inscription pattern to be inscribed inside or on the surface of any component of the lens. The apparatus can, in a second embodiment, also adjust at least one optical aberration of the optical sections of the lens which aberration can be pre-existing or which aberration is due to said mechanical adjustment.

So, this document discloses an apparatus with a digital control unit which is connected to at least one laser light source with the apparatus adapted to inscribe a pattern in any component of an intraocular lens with the apparatus providing inscription to adjust mechanical properties of at least one component of the lens, which component can be at least one haptic of the lens, or, alternatively, at least one optical component of the lens, which can be a shape changing optical component, with the apparatus adapted to either providing adjustment of the lens in any position outside the eye, or, alternatively, providing adjustment of the lens in any position outside the eye, with the apparatus comprising any laser light source, preferably a femto-second laser light source, with the apparatus providing inscription in the form of local ablation, or, local swelling, or, local inscription, or local cutting and with the apparatus comprising at least one diagnostic unit which is adapted to provide real-time input to the digital control unit, which diagnostic unit can comprise a light source and a detector which measures the optical properties of the lens which unit can comprise a mirror located in the optical path between the light source, the detector respectively and the lens, with the mirror, which can be a beam splitting mirror, located in the optical path between the laser light source and the lens, with the apparatus adapted to function according to a method to adjust mechanical properties of a haptic of an intraocular lens by providing an inscription pattern in the lens by a laser beam under control of a digital control unit which pattern is adapted to adjust mechanical properties of at least one component of the lens which component can be at least one haptic component of the lens, or, alternatively, at least one optical component of the lens, or, alternatively, a combination of any components.

## Claims

1. Apparatus comprising a digital control unit connected to at least one laser light source with the apparatus adapted to inscribe a pattern in any component of an intraocular lens **characterized in that** the apparatus is adapted to provide inscription adapted to adjust mechanical properties of at least one component of the lens.

2. Apparatus according to Claim 1 lens **characterized in that** the at least one component of the lens is at least one haptic of the lens.

3. Apparatus according to Claim 1 lens **characterized in that** the at least one component of the lens is at least one optical component of the lens.

4. Apparatus as claimed in any of the preceding claims, **characterized in that** the apparatus is adapted to adjust the properties of an intraocular lens in any position outside the eye.

5. Apparatus as claimed in any of the claims 1-3, **characterized in that** the apparatus is adapted to adjust the properties of an intraocular lens inside the eye.

6. Apparatus according to any of the preceding claims, **characterized in that** the laser light source is a femto-second laser light source.

7. Apparatus according to any of the preceding claims, **characterized in that** the apparatus is adapted to cause local ablation, or, local swelling, or, local inscription, or local cutting.

8. Apparatus according to any of the claims 4-7, **characterized in that** the apparatus
comprises a diagnostic unit which is adapted to provide real-time input to the digital control
unit.

9. Apparatus as claimed in claim 8, **characterized in that** the diagnostic unit comprises a light source and a detector which measures the optical properties of the lens.

10. Apparatus as claimed in claim 9, **characterized in that** the diagnostic unit comprises a mirror located in the optical path between the light source, the detector respectively and the lens, and that the mirror is located in the optical path between the laser light source and the lens.

11. Apparatus as claimed in claim 10, **characterized in that** the mirror is a beam splitting mirror.

12. Method to adjust mechanical properties of a haptic of an intraocular lens **characterized by** providing an inscription pattern in the lens by a laser beam under control of a digital control unit which pattern is adapted to adjust mechanical properties of at least one component of the lens.

13. Method as claimed in claim 12, **characterized by** providing the inscription pattern to at least one haptic component of the lens.

14. Method as claimed in claim 12, **characterized by** providing the inscription pattern to at least one optical component of the lens.
